(19) Europäisches Patentamt
European Patent Office
Office européen des brevets

(11) EP 0 847 999 B1

(12) **FASCICULE DE BREVET EUROPEEN**

(45) Date de publication et mention de la délivrance du brevet:
**21.03.2001 Bulletin 2001/12**

(51) Int Cl.[7]: **C07D 295/08**, C07D 311/66, C07D 311/58, A61K 31/495

(21) Numéro de dépôt: **97402934.0**

(22) Date de dépôt: **04.12.1997**

(54) **Dérivés de N-benzylpipérazine, leur procédé de préparation et les compositions pharmaceutiques qui les contiennent**

N-benzylpiperazinderivate, Verfahren zu ihrer Herstellung und sie enthaltende pharmazeutische Zubereitungen

N-benzylpiperazine derivatives, process for their preparation and pharmaceutical compositions containing them

(84) Etats contractants désignés:
**AT BE CH DE DK ES FI FR GB GR IE IT LI LU NL PT SE**

(30) Priorité: **16.12.1996 FR 9615415**

(43) Date de publication de la demande:
**17.06.1998 Bulletin 1998/25**

(73) Titulaire: **ADIR ET COMPAGNIE**
**92415 Courbevoie Cédex (FR)**

(72) Inventeurs:
- **Wierzbicki, Michel**
  **78620 L'Etang la Ville (FR)**
- **Boussard, Marie-Françoise**
  **78124 Mareil sur Mauldre (FR)**
- **Labidalle, Serge**
  **31120 Pinsaguel (FR)**
- **Guyot, Daniel**
  **31380 Montjoire (FR)**
- **Rolland, Yves**
  **92170 Vanves (FR)**
- **Tillement, Jean-Paul**
  **77590 Bois le Roi (FR)**
- **Testa, Bernard**
  **1005 Lausanne (CH)**
- **Crevat, Aimé**
  **13001 Marseille (FR)**

(56) Documents cités:
**EP-A- 0 326 379**
**EP-A- 0 617 027**
**FR-A- 1 302 958**
**FR-A- 1 303 080**
**FR-M- 805**
**FR-M- 1 549**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

EP 0 847 999 B1

## Description

La présente invention concerne de nouveaux dérivés de N-benzylpipérazine, leur procédé de préparation ainsi que les compositions pharmaceutiques qui les contiennent.

L'état antérieur de la technique est illustré notamment par :

- les brevets français 1 302 958 et 805 M qui concernent respectivement la préparation de N-trialkoxy benzyl pipérazines et l'utilisation comme médicament à action vaso-dilatatrice de la 2,3,4-triméthoxybenzyl pipérazine,
- les articles d'Hiroshi Ohtaka et coll., Chem. Pharm. Bull., 35, 2774-3275 (1987) et Chem. Pharm. Bull., 37, 11, 2124-3122 (1989) qui mentionnent des dérivés de trimétazidine ayant une activité vasodilatatrice, et la synthèse de 1-[bis(4-fluorophényl)méthyl]-4-(2-hydroxy 3,4-diméthoxybenzyl)pipérazine,
- l'article de Tsunéo Kawashima et coll., J. Pharmacobio-Dyn, 14, 449-459 (1991) relatif à l'isolation et l'identification de nouveaux métabolites du KB-2796 dont entre autres la 1-[bis(4-fluorophényl)méthyl]-4-(2-hydroxy 3,4-diméthoxybenzyl)pipérazine,
- le brevet européen EP 533 579 qui décrit des dérivés de N-benzylpipérazines ayant une activité antihypoxique et antiischémique,
- enfin, le brevet européen EP 617 027 qui décrit des dérivés de N-benzylpipérazines utiles pour le traitement des maladies neuronales dues au dysfonctionnement du métabolisme oxydatif.

Les dérivés de la présente invention, outre le fait qu'ils soient nouveaux, présentent une activité pharmacologique et des propriétés thérapeutiques particulièrement intéressantes.

Ils permettent, entre autres, la protection des mitochondries subissant un stress hypoxique, la restauration de la synthèse d'ATP par des organes privés d'oxygène et la protection d'un coeur isolé placé en situation d'ischémie. Enfin, ils sont capables de franchir la barrière hémato-encéphalique. Ces propriétés les rendent donc utiles pour le traitement de l'ischémie cellulaire chronique, des accidents ischémiques aigus cérébraux, cardiaques ou périphériques, pour le traitement des maladies neurodégénératives chroniques (comme la maladie d'Alzheimer ou la maladie de Parkinson) ainsi que pour l'amélioration de la conservation des organes destinés à des transplants et la survie des greffes lors du stress de reperfusion.

Plus spécifiquement, la présente invention concerne les composés de formule (I) :

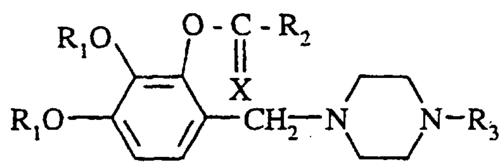

(I)

dans laquelle:

- $R_1$ représente un groupement alkyle ($C_1$-$C_6$) linéaire ou ramifié,
- X représente un atome d'oxygène ou de soufre,
- $R_2$ représente un groupement alkyle ($C_1$-$C_8$) linéaire ou ramifié (éventuellement substitué par un groupement carboxy ou alkoxycarbonyle ($C_1$-$C_6$) linéaire ou ramifié), un groupement alkoxy ($C_1$-$C_6$) linéaire ou ramifié, un groupement phényle (éventuellement substitué par un ou plusieurs atomes d'halogène ou groupements alkyle ($C_1$-$C_6$) linéaire ou ramifié, alkoxy ($C_1$-$C_6$) linéaire ou ramifié, hydroxy ou trihalogénométhyle), un groupement cycloalkyle ($C_3$-$C_7$) (éventuellement substitué par un ou plusieurs groupements phényle eux-mêmes éventuellement substitués par un ou plusieurs atomes d'halogène ou groupements alkyle ($C_1$-$C_6$) linéaire ou ramifié, alkoxy ($C_1$-$C_6$) linéaire ou ramifié, hydroxy, ou trihalogénométhyle), un groupement 4-(2,3-dithiacyclopent-l-yl)butyl, un groupement pyridyle, un groupement amino (éventuellement substitué par un ou deux groupements alkyle ($C_1$-$C_6$) linéaire ou ramifié), ou l'un quelconque des groupements suivants :

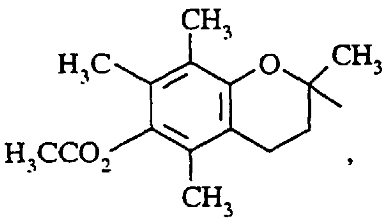
,
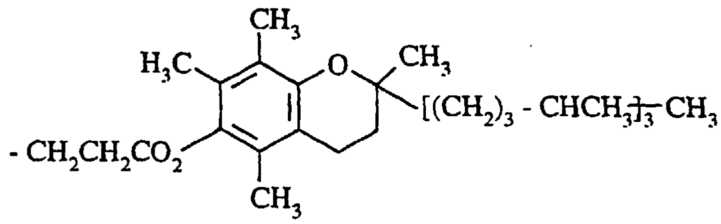

$R_3$ représente un atome d'hydrogène, un groupement cycloalkyle ($C_3$-$C_7$), un groupement formyle, un groupement phényle (éventuellement substitué par un ou plusieurs atomes d'halogène ou groupements alkyle ($C_1$-$C_6$) linéaire ou ramifié, alkoxy ($C_1$-$C_6$) linéaire ou ramifié, hydroxy ou trihalogénométhyle), un groupement pyridyle,

ou, un groupement alkyle ($C_1$-$C_{20}$) linéaire ou ramifié éventuellement substitué par un ou plusieurs, identiques ou différents, atomes d'halogène ou groupements :

- phényle éventuellement substitué par un ou plusieurs atomes d'halogène ou groupements alkyle ($C_1$-$C_6$) linéaire ou ramifié, alkoxy ($C_1$-$C_6$) linéaire ou ramifié, hydroxy ou trihalogénométhyle,
- cycloalkyle ($C_3$-$C_7$) éventuellement substitué par un ou plusieurs groupements phényle eux-mêmes éventuellement substitués par un ou plusieurs atomes d'halogène ou groupements alkyle ($C_1$-$C_6$) linéaire ou ramifié, alkoxy ($C_1$-$C_6$) linéaire ou ramifié, hydroxy ou trihalogénométhyle,
- alkoxy ($C_1$-$C_6$) linéaire ou ramifié,
- hydroxy,
- ou, pyrolidinyle, leurs stéréoisomères lorsqu'ils existent ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...
Parmi les bases pharmaceutiquement acceptables, on peut citer à titre non limitatif l'hydroxyde de sodium, l'hydroxyde de potassium, la triéthylamine, la tertbutylamine, etc...
L'invention s'étend également au procédé de préparation des composés de formule (I) caractérisé en ce que l'on utilise comme produit de départ un composé de formule (II) :

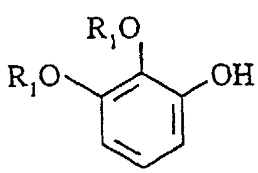
(II)

dans laquelle $R_1$ a la même signification que dans la formule (I), que l'on fait réagir avec une pipérazine substituée de formule (III):

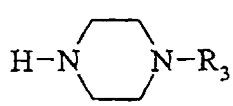
(III)

dans laquelle $R_3$ a la même signification que dans la formule (I), en présence de formaldéhyde,
pour conduire au composé de formule (IV) ;

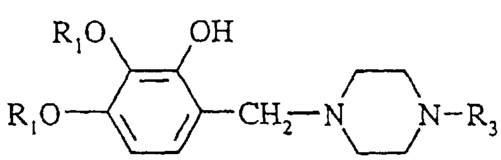
(IV)

dans laquelle $R_1$ et $R_3$ on la même signification que dans la formule (I),
que l'on fait ensuite réagir avec un acide carboxylique de formule $R_2CO_2H$ (dans laquelle $R_2$ a la même signification que dans la formule (I), un chloroformiate d'alkyle, un chlorure d'acide de formule $R_2COCl$ (dans laquelle $R_2$ a la même signification que précédemment), un anhydride d'acide, ou un chlorocarbamate,
pour conduire au composé de formule (I/a), cas particulier des composés de formule (I) :

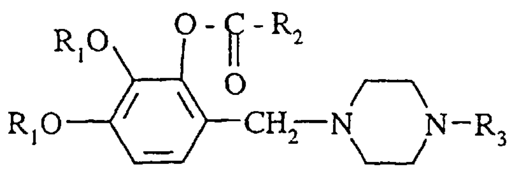

(I/a)

dans laquelle $R_1$, $R_2$ et $R_3$ ont la même signification que précédemment,
composé de formule (I/a) que l'on soumet éventuellement à l'action du réactif de Lawesson, pour conduire au thioester correspondant de formule (I/b), cas, particulier des composés de formule (I):

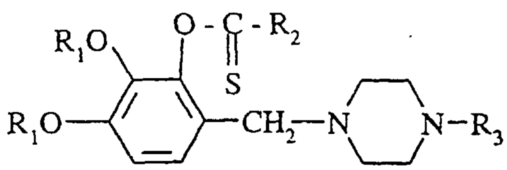

(I/b)

dans laquelle $R_1$, $R_2$ et $R_3$ ont la même signification que dans la formule (I),
composés de formule (I/a) ou (I/b),
que l'on purifie, le cas échéant, selon une technique classique de purification, dont on sépare éventuellement les stéréoisomères lorsqu'ils existent, et que l'on transforme, si on le souhaite, en sel d'addition à un acide ou une base pharmaceutiquement acceptable.

Les composés de l'invention dans lesquels $R_3$ représente un atome d'hydrogène sont plus particulièrement obtenus à partir du composé de formule (I/a) dans lequel $R_3$ représente un groupement benzyle que l'on soumet à une hydrogénation catalytique dans le diméthylformamide.

Les composés préférés de l'invention sont les composés de formule (I) dans lesquels $R_1$ représente un groupement méthyle et X représente un atome d'oxygène.

Parmi les composés préférés de l'invention pour lesquels $R_1$ représente un groupement méthyle et X un atome d'oxygène, le substituant $R_2$ préféré est le groupement cycloalkyle ($C_3$-$C_7$) éventuellement substitué et plus particulièrement le groupement phénylcyclopropyle. Le substituant $R_3$ préféré est le groupement alkyle éventuellement substitué et plus particulièrement les groupements éthyle ou benzyle.

La présente invention a également pour objet les compositions pharmaceutiques renfermant comme principe actif au moins un composé de formule (I) seul ou en combinaison avec un ou plusieurs excipients ou véhicules inertes non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 0,1 et 500 mg pour un traitement en 1 à 3 prises par 24 heures.

Les exemples suivants illustrent l'invention mais ne la limitent en aucune façon.

Les produits de départ utilisés sont des produits connus ou préparés selon des modes opératoires connus.

Les structures des composés décrits dans les exemples ont été déterminées selon les techniques spectrophotométriques usuelles (infrarouge, RMN, spectrométrie de masse ...).

***EXEMPLE 1*: N-(3,4-Diméthoxy-2-E-phénylcyclopropylcarbonyloxy)benzyl-N'-éthyl-pipérazine, dichlorhydrate**

**Stade A : N-(3,4-Diméthoxy-2-hydroxy)benzyl-N'-éthyl-pipérazine**

On additionne simultanément dans un réacteur 56 mmoles de N-éthylpipérazine dans 100 ml d'éthanol et 56

mmoles de 2,3-diméthoxyphénol dans 100 ml d'éthanol en agitant vigoureusement à température ambiante. A cette solution est ajoutée goutte à goutte 45 ml d'une solution aqueuse de formaldéhyde à 38 % P/V. Le milieu réactionnel est agité 24 heures à température ambiante puis est évaporé à sec sous vide partiel.
Le résidu obtenu est chromatographié sur gel de silice avec un mélange éluant de dichlorométhane/méthanol (97/3) et conduit au produit attendu.

**<u>Stade B</u> : N-(3,4-Diméthoxy-2-E-phénylcyclopropylcarbonyloxy)benzyl-N'-éthyl-pipérazine, dichlorhydrate**

A une solution de 40 mmoles d'acide 2-E-phénylcyclopropylcarboxylique dans un mélange de dichlorométhane et de diméthylformamide (90/10) maintenue à 0°C sous atmosphère inerte, on ajoute goutte à goutte, sous agitation, une solution de 120 mmoles de chlorure d'oxalyle dans 20 ml de dichlorométhane. Le milieu réactionnel est maintenu à 0°C pendant une heure puis à 25°C pendant 12 heures. L'excès de chlorure d'oxalyle et le solvant sont évaporés sous vide partiel et le résidu obtenu est repris dans 20 ml de dichlorométhane.
La solution ainsi obtenue est additionnée goutte à goutte à une solution de 40 mmoles du composé obtenu au stade précédent dans 150 ml de pyridine anhydre. Le milieu réactionnel est agité à 25°C pendant 24 heures puis est évaporé à sec sous vide partiel.
Le résidu obtenu est chromatographié sur gel de silice avec un mélange éluant de dichlorométhane/méthanol (95/5) et conduit au produit attendu sous forme de base. Ce composé est dissous dans le minimum d'éther anhydre puis est additionné à une quantité deux fois stoechiométrique d'HCl en solution dans l'éther anhydre. Un précipité abondant se forme aussitôt. On le recueille par filtration et on le recristallise dans un mélange éthanol/éther. Le dichlorhydrate précipite, est filtré et recristallisé dans un mélange éthanol/éther.

| *Microanalyse élémentaire* : | | | |
|---|---|---|---|
| | *C%* | *H%* | *N%* |
| *calculée* | *60, 36* | *6,89* | *5,63* |
| *trouvée* | *60,30* | *6,89* | *5,65* |

*<u>Point de fusion</u> : 222°C*

***<u>EXEMPLE 2</u> : N-(3,4-Diméthoxy-2-E.phénylcyclopropylcarlbonyloxy)benzyl -N'-benzyl-pipérazine, dichlorhydrate***

Le produit attendu est obtenu selon le procédé décrit dans l'exemple 1 en remplaçant au stade A la N-éthylpipérazine par la N-benzylpipérazine.

| *Microanalyse élémentaire :* | | | |
|---|---|---|---|
| | *C%* | *H%* | *N%* |
| *calculée* | *62,39* | *6,63* | *4,85* |
| *trouvée* | *62,23* | *6,34* | *4,97* |

*<u>Point de fusion</u> : 216°C*

***<u>EXEMPLE 3</u> : N-(2-Benzoyloxy-3,4-diméthoxy)benzyl-N'-benzyl-piérazine, dichlorhydrate***

Le produit attendu est obtenu selon le procédé décrit dans l'exemple 1 en remplaçant au stade A la N-éthylpipérazine par la N-benzylpipérazine et au stade B l'acide 2-E-phénylcyclopropylcarboxylique par l'acide benzoïque.

| *Microanalyse élémentaire* : | | | |
|---|---|---|---|
| | *C%* | *H%* | *N%* |
| *calculée* | *62,43* | *6,21* | *5,39* |
| *trouvée* | *62, 85* | *6,18* | *5,39* |

*<u>Point de fusion</u> : 210°C*
Les exemples suivants ont été préparés selon le procédé décrit dans l'exemple 1 en utilisant les produits de

départ correspondants.

***EXEMPLE 4*: N-(2-Benzoyloxy-3,4-diméthoxy)benzyl-N'-éthyl-pipérazine, dichlorhydrate**

| *Microanalyse élémentaire :* | | | |
|---|---|---|---|
| | *C%* | *H%* | *N%* |
| *calculée* | *57, 77* | *6,61* | *6,12* |
| *trouvée* | *57,97* | *6,80* | *6,42* |

*Point de fusion : 205°C (dec.)*

***EXEMPLE 5* : N-(2-Acétoxy-3,4-diméthoxy)benzyl-N'-éthyl-pipérazine, dichlorhydrate**

| *Microanalyse élémentaire :* | | | |
|---|---|---|---|
| | *C%* | *H%* | *N%* |
| *calculée* | *51,65* | *7,14* | *7, 09* |
| *trouvée* | *51,55* | *6,95* | *7,32* |

*Point de fusion : 230°C(dec.)*

***EXEMPLE 6* : N-(2-Acétoxy-3,4-diméthoxy)benzyl-N'-benzyl-pipérazine, dichlorhydrate**

| *Microanalyse élémentaire :* | | | |
|---|---|---|---|
| | *C%* | *H%* | *N%* |
| *calculée* | *57, 77* | *6,61* | *6,12* |
| *trouvée* | *58,05* | *6,57* | *6,10* |

*Point de fusion : 220°C (dec.)*

***EXEMPLE 7*: N-(2-tert-Butylcarbonyloxy-3,4-diméthoxy)benzyl-N'-éthyl-pipérazine, dichlorhydrate**

***EXEMPLE 8* : N-(2-tert-Butylcarbonyloxy-3,4-diméthoxy)benzyl-N'-benzyl-pipérazine, dichlorhydrate**

***EXEMPLE 9*: N-(3,4-Diméthoxy-2-octanoyloxy)benzyl-N'-éthyl-pipérazine, dichlorhydrate**

***EXEMPLE 10*: N-(3,4-Diméthoxy-2-octanoyloxy)benzyl-N'-benzyl-piérazine, dichlorhydrate**

***EXEMPLE 11*: N-(3,4-Diméthoxy-2-nicotinoyloxy)benzyl-N'-éthyl-pipérazine, trichlorhydrate**

| *Microanalyse élémentaire :* | | | |
|---|---|---|---|
| | *C %* | *H%* | *N%* |
| *calculée* | *50,97* | *6,11* | *8,49* |
| *trouvée* | *50, 44* | *6,24* | *8,26* |

*Point de fusion: 180°C*

***EXEMPLE 12: N-(3,4-Diméthoxy-2-nicotinoyloxy)benzyl-N'-benzyl-pipérazine, trichlorhydrate***

| Microanalyse élémentaire : | | | |
|---|---|---|---|
| | C % | H% | N% |
| calculée | 56,07 | 5, 79 | 7,55 |
| trouvée | 56,19 | 5,53 | 7,56 |

*Point de fusion: 213°C*

***EXEMPLE 13: N-[2-(6'-Acétoxy-2',5',7',8'-tétraméthylchroman-2-yl-carbonyloxy)-3,4-diméthoxy]benzyl-N'-éthyl-pipérazine, dichlorhydrate***

| Microanalyse élémentaire : | | | |
|---|---|---|---|
| | C% | H% | N% |
| calculée | 59, 33 | 7,07 | 4,46 |
| trouvée | 58, 97 | 7,10 | 4,18 |

*Point de fusion: 215°C*

***EXEMPLE 14: N-(2-(6'-Acétoxy-2',5',7',8'-tétraméthylchroman-2-yl-carbonyloxy)-3,4-diméthozy]benzyl-N'-benzyl-pipérazine, dichlorhydrate***

| Microanalyse élémentaire : | | | |
|---|---|---|---|
| | C% | H% | N% |
| calculée | 62, 70 | 6, 72 | 4, 06 |
| trouvée | 62,58 | 6,97 | 4,27 |

*Point de fusion: 227°C*

***EXEMPLE 15: N-{2-[5-(2,3-dithiacyclopent-1-yl)propanoyloxy-3,4-diméthoxy}benzyl-N'-éthyl-pipérazine, dichlorhydrate***

| Microanalyse élémentaire : | | | |
|---|---|---|---|
| | C% | H% | N% |
| calculée | 51,01 | 7,07 | 5,17 |
| trouvée | 50,67 | 6, 71 | 4, 78 |

*Point de fusion: 204°C*

***EXEMPLE 16**: N-{2-[5-(2,3-dithiacyclopent-1-yl)propanoyloxy-3,4-diméthoxy}benzyl-N'-benzyl-pipérazine, dichlorhydrate*

| *Microanalyse élémentaire :* | | | |
|---|---|---|---|
| | *C%* | *H%* | *N%* |
| *calculée* | *55,71* | *6, 68* | *4, 64* |
| *trouvée* | *55, 92* | *6,52* | *4,81* |

*Point de fusion: 220°C (dec.)*

***EXEMPLE 17**: N-(3,4-Diméthoxy-2-E-(R,R)-phénylcyclopropylcarbonyloxy)benzyl-N'-éthyl-pipérazine, dichlorhydrate*

| *Microanalyse élémentaire :* | | | |
|---|---|---|---|
| | *C%* | *H%* | *N%* |
| *calculée* | *60, 36* | *6, 89* | *5,63* |
| *trouvée* | *60, 30* | *6,89* | *5,65* |

*Point de fusion: 200°C*

***EXEMPLE 18**: N-(3,4-Diméthoxy-2-E-(R,R)-phénylcyclopropylcarbonyloxy)benzyl-N'-benzyl-pipérazine, dichlorhydrate*

| *Microanalyse élémentaire :* | | | |
|---|---|---|---|
| | C% | *H%* | *N%* |
| *calculée* | *64,40* | *6,49* | *5,01* |
| *trouvée* | *64,35* | *6, 76* | *4,82* |

*Point de fusion: 206°C*

***EXEMPLE 19**: N-(3,4-Diméthoxy-2-E-(S,S)-phénylcyclopropylcarbonyloxy)benzyl-N'-éthyl-pipérazine, dichlorhydrate*

| *Microanalyse élémentaire :* | | | |
|---|---|---|---|
| | *C%* | *H%* | *N%* |
| *calculée* | *60, 36* | *6,89* | *5,63* |
| *trouvée* | *60,30* | *6,89* | *5,65* |

*Point de fusion: 210°C*

***<u>EXEMPLE 20</u>: N-(3,4-Diméthoxy-2-E-(S,S)-phénylcyclopropylcarbonyloxy)benzyl-N'-benzyl-pipérazine, dichlorhydrate***

| Microanalyse élémentaire : | | | |
|---|---|---|---|
| | C% | H% | N% |
| calculée<br>trouvée | 64, 40<br>64,25 | 6,49<br>6,32 | 5,01<br>5,23 |

*<u>Point de fusion</u>: 205°C*

***<u>EXEMPLE 21</u>: N-[3,4-Diméthoxy-2-(3-carboxypropionyloxy)]benzyl-N'-éthyl-pipérazine, dichlorhydrate***

***<u>EXEMPLE 22</u>: N-(3,4-Diméthoxy-2-(3-carboxypropionyloxy)]benzyl-N'-benzyl-pipérazine, dichlorhydrate***

***<u>EXEMPLE 23</u>: N-(3,4-Diméthoxy-2-(7-méthoxycarbonylheptanoyloxy)]benzyl-N'-éthyl-pipérazine, dichlorhydrate***

***<u>EXEMPLE 24</u>: N-3,4-Diméthoxy-2-[7-méthoxycarbonylheptanoyloxy]benzyl-N'-benzyl-pipérazine, dichlorhydrate***

***<u>EXEMPLE 25</u>: N-(3,4-Diméthoxy-2-(3-tocophéroylpropionyloxy)]benzyl-N'-éthyl-pipérazine, dichlorhydrate***

Tocophéroyl représente le groupement :

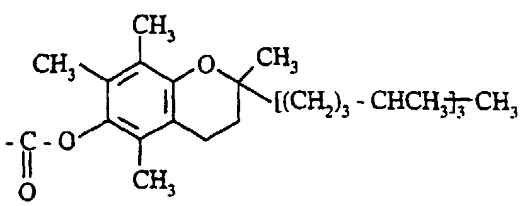

| Microanalyse élémentaire : | | | |
|---|---|---|---|
| | C% | H% | N% |
| calculée<br>trouvée | 66,57<br>66,42 | 9,08<br>9,35 | 3,23<br>3,50 |

*<u>Point de fusion</u>: 190°C*

***<u>EXEMPLE 26</u>: N-(3,4-Diméthoxy-2-(3-tocophéroylpropionyloxy)]benzyl-N'-benzyl-pipérazine, dichlorhydrate***

| Microanalyse élémentaire : | | | |
|---|---|---|---|
| | C% | H% | N% |
| calculée<br>trouvée | 68, 59<br>68, 32 | 8,69<br>8,92 | 3,02<br>3,24 |

*<u>Point de fusion</u>: 206°C*

***EXEMPLE 27**: N-(2-Ethoxycarbonyloxy-3,4-diméthoxy)benzyl-N'-benzyl-pipérazine, dichlorhydrate*

A une solution de 10 mmoles de N-benzyl-N'-(3,4-diméthoxy-2-hydroxy)benzyl-pipérazine dans 100 ml de dichlorométhane anhydre, on ajoute goutte à goutte 11 mmoles d'une solution de chloroformiate d'éthyle dans 10 ml de dichlorométhane. Après agitation à température ambiante pendant 24 h, on observe la formation d'un précipité. Après neutralisation par une solution aqueuse de carbonate de sodium, le milieu réactionnel est extrait par le dichlorométhane et la phase organique est concentrée à sec sous vide partiel et le résidu est chromatographié sur colonne de silice (éluant : dichlorométhane/méthanol : 95/5). Les fractions retenues sont réunies puis concentrées sous vide partiel.
10 mmoles du composé ainsi obtenu dissoutes dans le minimum d'éther anhydre sont additionnées à une quantité deux fois stoechiométrique d'HCl en solution dans l'éther anhydre. Un précipité abondant se forme aussitôt, est filtré et recristallisé dans un mélange éthanol/éther et conduit au produit attendu.

| *Microanalyse élémentaire :* | | | |
|---|---|---|---|
| | *C%* | *H%* | *N%* |
| *calculée* | *56,68* | *6,62* | *5,75* |
| *trouvée* | *56,42* | *6,85* | *5,42* |

*Point de fusion: 223°C*

***EXEMPLE 28**: N-(2-Ethoxycarbonyloxy-3,4-diméthoxy)benzyl-N'éthyl-pipérazine, dichlorhydrate*

Le produit attendu est obtenu selon le procédé décrit dans l'exemple 27.

| *Microanalyse élémentaire :* | | | |
|---|---|---|---|
| | *C%* | *H%* | *N%* |
| *calculée* | *50, 83* | *7,11* | *6,59* |
| *trouvée* | *50, 62* | *6, 96* | *6,15* |

*Point de fusion: 213°C*

***EXEMPLE 29**: N-(2-Diméthylaminocarbonyloxy-3,4-diméthoxy)benzyl-N'-benzyl-pipérazine, dichlorhydrate*

A une solution de 10 mmoles de N-benzyl-N'-(3,4-diméthoxy-2-hydroxy)benzyl-pipérazine dans 100 ml de dichlorométhane anhydre, on ajoute goutte à goutte il mmoles d'une solution de chlorure de N,N-diméthylcarbamoyle dans 10 ml de dichlorométhane. Après agitation à température ambiante pendant 24 h, on observe la formation d'un précipité. Après neutralisation par une solution aqueuse de carbonate de sodium, le milieu réactionnel est extrait par le dichlorométhane et la phase organique est concentrée à sec sous vide partiel et le résidu chromatographié sur colonne de silice (éluant : dichlorométhane/méthanol : 95/5). Les fractions retenues sont réunies puis concentrées sous vide partiel et conduisent au produit attendu.

| *Microanalyse élémentaire :* | | | |
|---|---|---|---|
| | *C%* | *H%* | *N%* |
| *calculée* | *58,36* | *7,25* | *8,17* |
| *trouvée* | *58,59* | *6,98* | *8,26* |

*Point de fusion: 229°C*

*EXEMPLE 30 : N-(2-Diméthylaminocarbonyloxy-3,4-diméthoxy)benzyl-N'-éthyl-pipérazine, dichlorhydrate*

Le produit attendu est obtenu selon le procédé décrit dans l'exemple 29.

| *Microanalyse élémentaire :* | | | |
|---|---|---|---|
| | *C%* | *H%* | *N%* |
| *calculée* | *53,10* | *7,80* | *9,29* |
| *trouvée* | *53, 25* | *7,52* | *9,66* |

*Point de fusion : 190°C*

Les exemples suivants ont été préparés selon le procédé de l'exemple 1 à partir des produits de départ correspondants.

***EXEMPLE 31 : N-(3,4-Diméthoxy-2-E-phénylcyclopropylcarbonyloxy)benzyl-N'-formyl-pipérazine, chlorhydrate***

***EXEMPLE 32 : N-(3,4-Diméthoxy-2-benzoyloxy)benzyl-N'-formyl-pipérazine, chlorhydrate***

***EXEMPLE 33 : N-(3,4-Diméthoxy-2-E-(S,S)-phénylcyclopropylcarbonyloxy)benzyl-N'-formyl-pipérazine, chlorhydrate***

***EXEMPLE 34 : N-(3,4-Diméthoxy-2-E-(R,R)-phénylcyclopropylcarbonyloxy)benzyl-N'-formyl-pipérazine, chlorhydrate***

***EXEMPLE 35 : N-(3,4-Diméthoxy-2-E-phénylcyclopropylcarbonyloxy)benzyl-N'-cyclohexyl-pipérazine, dichlorhydrate***

***EXEMPLE 36 : N-(3,4-Diméthoxy-2-benzoyloxy)benzyl-N'-cyclohexyl-pipérazine, dichlorhydrate***

***EXEMPLE 37 : N-(3,4-Diméthoxy-2-E-(S,S)-phénylcyclopropylcarbonyloxy)benzyl-N'-cyclohexyl-pipérazine, dichlorhydrate***

***EXEMPLE 38 : N-(3,4-Diméthoxy-2-E-(R,R)-phénylcyclopropylcarbonyloxy)benzyl-N'-cyclohexyl-pipérazine, dichlorhydrate***

***EXEMPLE 39 : N-(3,4-Diméthoxy-2-E-phénylcyclopropylcarbonyloxy)benzyl-N'-phényl-pipérazine, dichlorhydrate***

***EXEMPLE 40 : N-(3,4-Diméthoxy-2-benzoyloxy)benzyl-N'-phényl-pipérazine, dichlorhydrate***

***EXEMPLE 41 : N-(3,4-Dimédioxy-2-E-(S,S)-phénylcyclopropylcarbonyloxy)benzyl-N'-phényl-pipérazine, dichlorhydrate***

***EXEMPLE 42 : N-(3,4-Diméthoxy-2-E-(R,R)-phénylcyclopropylcarbonyloxy)benzyl-N'-phényl-pipérazine, dichlorhydrate***

***EXEMPLE 43 : N-(3,4-Diméthoxy-2-E-phénylyclopropylcarbonyloxy)benzyl-N'-2-pyridinyl-pipérazine, trichlorhydrate***

***EXEMPLE 44 : N-(3,4-Diméthoxy-2-benzoyloxy)benzyl-N'-2-pyridinyl-pipérazine, trichlorhydrate***

***EXEMPLE 45 : N-(3,4-Diméthoxy-2-E-(S,S)-phénylcyclopropylcarbonyloxy)benzyl-N'-2-pyridinyl-pipérazine, trichlorhydrate***

***EXEMPLE 46 : N-(3,4-Diméthoxy-2-E-(R,R)-phénylcyclopropylcarbonyloxy)benzyl-N'-2-pyridinyl-pipérazine, trichlorhydrate***

***EXEMPLE 47 : N-(3,4-Diméthoxy-2-E-phénylcyclopropylcarbonyloxy)benzyl-N'-méthyl-pipérazine, dichlorhydrate***

***EXEMPLE 48**: N-(3,4-Diméthoxy-2-benzoyloxy)benzyl-N'-méthyl-pipérazine, dichlorhydrate*

***EXEMPLE 49**: N-(3,4-Diméthoxy-2-E-(S,S)-phénylcyclopropylcarbonyloxy)benzyl-N'-méthyl-pipérazine, dichlorhydrate*

***EXEMPLE 50** : N-(3,4-Diméthoxy-2-E-(R,R)-phénylcyclopropylcarbonyloxy)benzyl-N'-méthyl-pipérazine, dichlorhydrate*

***EXEMPLE 51** : N-(3,4-Diméthoxy-2-E-phénylcyclopropylcarbonyloxy)benzyl-N'-4,4'-difluorobenzhydryl-pipérazine, dichlorhydrate*

***EXEMPLE 52** : N-(3,4-Diméthoxy-2-benzoyloxy)benzyl-N'-4,4'-difluorobenzhydryl-pipérazine, dichlorhydrate*

***EXEMPLE 53** : N-(3,4-Diméthoxy-2-E-(S,S)-phénylcyclopropylcarbonyloxy)benzyl-N'-4,4'-difluorobenzhydryl-pipérazine, dichlorhydrate*

***EXEMPLE 54** : N-(3,4-Diméthoxy-2-E-(R,R)-phénylcyclopropylcarbonyloxy)benzyl-N'-4,4'-difluorobenzhydryl-pipérazine, dichlorhydrate*

***EXEMPLE 55** : N-(3,4-Diméthoxy-2-E-phénylcyclopropylcarbonyloxy)benzyl-N'-benzhydryl-pipérazine, dichlorhydrate*

***EXEMPLE 56**: N-(3,4-Diméthoxy-2-benzoyloxy)benzyl-N'-benzhydryl-pipérazine, dichlorhydrate*

***EXEMPLE 57** : N-(3,4-Diméthoxy-2-E-(S,S)-phénylcyclopropylcarbonyloxy)benzyl-N'-benzhydryl-pipérazine, dichlorhydrate*

***EXEMPLE 58** : N-(3,4-Diméthoxy-2-E-(R,R)-phénylcyclopropylcarbonyloxy)benzyl-N'-benzhydryl-pipérazine, dichlorhydrate*

***EXEMPLE 59** : N-(3,4-Diméthoxy-2-E-phénylcyclopropylcarbonyloxy)benzyl-N'-[2-(N-pyrolidinyl)éthyl]-pipérazine, trichlorhydrate*

***EXEMPLE 60** : N-(3,4-Diméthoxy-2-benzoyloxy)benzyl-N'-[2-(N-pyrolidinyl)éthyl]-pipérazine, trichlorhydrate*

***EXEMPLE 61** : N-(3,4-Diméthoxy-2-E-(S,S)-phénylcyclopropylcarbonyloxy)benzyl-N'-[2-(N-pyrolidinyl)éthyl]-pipérazine, trichlorhydrate*

***EXEMPLE 62** : N-(3,4-Diméthoxy-2-E-(R,R)-phénylcyclopropylcarbonyloxy)benzyl-N'-[2-(N-pyrolidinyl)éthyl]-pipérazine, trichlorhydrate*

***EXEMPLE 63** : N-(3,4-Diméthoxy-2-E-phénylcyclopropylcarbonyloxy)benzyl-N'-(4-fluorobenzyl)-pipérazine, dichlorhydrate*

***EXEMPLE 64** : N-(3,4-Diméthoxy-2-benzoyloxy)benzyl-N'-(4-fluorobenzyl)-pipérazine, dichlorhydrate*

***EXEMPLE 65** : N-(3,4-Diméthoxy-2-E-(S,S)-phénylcyclopropylcarbonyloxy)benzyl-N'-(4-fluorobenzyl)-pipérazine, dichlorhydrate*

***EXEMPLE 66** : N-(3,4-Diméthoxy-2-E-(R,R)-phénylcyclopropylcarbonyloxy)benzyl-N'-(4-fluorobenzyl)-pipérazine, dichlorhydrate*

***EXEMPLE 67** : N-(3,4-Diméthoxy-2-E-phénylcyclopropylcarbonyloxy)benzyl-N'-isopropyl-pipérazine, dichlorhydrate*

***EXEMPLE 68** : N-(3,4-Diméthoxy-2-benzoyloxy)benzyl-N'-isopropyl-pipérazine, dichlorhydrate*

***EXEMPLE 69** : N-(3,4-Diméthoxy-2-E-(S,S)-phénylcyclopropylcarbonyloxy)benzyl-N'-isopropyl-pipérazine, dichlorhydrate*

***EXEMPLE 70** : N-(3,4-Diméthoxy-2-E-(R,R)-phénylcyclopropylcarbonyloxy)benzyl-N'-isopropyl-pipérazine, dichlorhydrate*

***EXEMPLE 71** : N-(3,4-Diméthoxy-2-E-phénylcyclopropylcarbonyloxy)benzyl-N'-propyl-pipérazine, dichlorhydrate*

***EXEMPLE 72** : N-(3,4-Diméthoxy-2-benzoyloxy)benzyl-N'-propyl-pipérazine, dichlorhydrate*

***EXEMPLE 73** : N-(3,4-Diméthoxy-2-E-(S,S)-phénylcyclopropylcarbonyloxy)benzyl-N'-propyl-pipérazine, dichlorhydrate*

***EXEMPLE 74** : N-(3,4-Diméthoxy-2-E-(R,R)-phénylcyclopropylcarbonyloxy)benzyl-N'-propyl-pipérazine, dichlorhydrate*

***EXEMPLE 75**: N-(3,4-Diméthoxy-2-E-phénylcyclopropylcarbonyloxy)benzyl-N'-heptyl-pipérazine, dichlorhydrate*

***EXEMPLE 76** : N-(3,4-Diméthoxy-2-benzoyloxy)benzyl-N'-heptyl-pipérazine, dichlorhydrate*

***EXEMPLE 77**: N-(3,4-Diméthoxy-2-E-(S,S)-phénylcyclopropylcarbonyloxy)benzyl-N'-heptyl-pipérazine, dichlorhydrate*

***EXEMPLE 78** : N-(3,4-Diméthoxy-2-E-(R,R)-phénylcyclopropylcarbonyloxy)benzyl-N'-heptyl-pipérazine, dichlorhydrate*

***EXEMPLE 79** : N-(3,4-Diméthoxy-2-E-phénylcyclopropylcarbonyloxy)benzyl-N'-hexadécyl-pipérazine, dichlorhydrate*

***EXEMPLE 80** : N-(3,4-Diméthoxy-2-benzoyloxy)benzyl-N'-hexadécyl-pipérazine, dichlorhydrate*

***EXEMPLE 81** : N-(3,4-Diméthoxy-2-E-(S,S)-phénylcyclopropylcarbonyloxy)benzyl-N'-hexadécyl-pipérazine, dichlorhydrate*

***EXEMPLE 82** : N-(3,4-Diméthoxy-2-E-(R,R)-phénylcyclopropylcarbonyloxy)benzyl-N'-hexadécyl-pipérazine, dichlorhydrate*

***EXEMPLE 83** : N-(3,4-Diméthoxy-2-E-phénylcyclopropylcarbonyloxy)bentyl-N'-octadécyl-pipérazine, dichlorhydrate*

***EXEMPLE 84** : N-(3,4-Diméthoxy-2-benzoyloxy)benzyl-N'-octadécyl-pipérazine, dichlorhydrate*

***EXEMPLE 85** : N-(3,4-Diméthoxy-2-E-(S,S)-phénylcyclopropylcarbonyloxy)benzyl-N'-octadécyl-pipérazine, dichlorhydrate*

***EXEMPLE 86** : N-(3,4-Diméthoxy-2-E-(R,R)-phénylcyclopropylcarbonyloxy))benzyl-N'--octadécyl-pipérazine, dichlorhydrate*

***EXEMPLE 87** : N-(3,4-Diméthoxy-2-E-phénylcyclopropylcarbonyloxy)benzyl-N'-(3-chloropropyl)-pipérazine, dichlorhydrate*

***EXEMPLE 88** : N-(3,4-Diméthoxy-2-benzoyloxy)benzyl-N'-(3-chloropropyl)-pipérazine, dichlorhydrate*

***EXEMPLE 89** : N-(3,4-Diméthoxy-2-E-(S,S)-phénylcyclopropylcarbonyloxy)benzyl-N'-(3-chloropropyl)-pipérazine, dichlorhydrate*

***EXEMPLE 90*** ***: N-(3,4-Diméthoxy-2-E-(R,R)-phénylcyclopropylcarbonyloxy)benzyl-N'-(3-chloropropyl)-pipérazine, dichlorhydrate***

***EXEMPLE 91*** ***: N-(2-Acétoxy-3,4-diméthoxy)benzyl-N'-(3-méthyl-12-(4-méthylphényl)] dodécyl-pipérazine, dichlorhydrate***

***EXEMPLE 92*** ***: N-(2-Isobutyryloxy-3,4-diméthoxy)benzyl-N'-benzyl-pipérazine***

*Point de fusion : huile*

***EXEMPLE 93*** ***: N-(2-Isobutyryloxy-3,4-diméthoxy)benzyl-N'-formyl-pipérazine***

*Point de fusion : huile*

***EXEMPLE 94*** ***: N-(3,4-Diméthoxy-2-(nicotinoyloxy)benzyl-N'-trityl-pipérazine***

***EXEMPLE 95*** ***: N-(2-Isobutyryloxy-3,4-diméthoxy)benzyl-pipérazine***

Le composé attendu est obtenu par débenzylation du composé de l'exemple 92, dans le diméthylformamide sous pression d'hydrogène en présence du catalyseur, Pd/C à 5 %.

*Point de fusion : huile*

Les composés des exemples 96 à 99 ont été obtenus selon le procédé décrit dans l'exemple 95 et transformés en sels correspondants en milieu chlorhydrique.

***EXEMPLE 96*** ***: N-(2-tert-Butylcarbonyloxy-3,4-diméthoxy)benzyl-pipérazine, dichlorhydrate***

*Point de fusion : 230°C*

***EXEMPLE 97*** ***: N-(3,4-Diméthoxy-2-E-phénylcyclopropylcarbonyloxy)benzyl-pipérazine, dichlorhydrate***

***EXEMPLE 98*** ***: N-(2-Benzoyloxy-3,4-diméthoxy)benzyl-pipérazine, dichlorhydrate***

***EXEMPLE 99*** ***: N-(3,4-Diméthoxy-3-nicotinoyloxy)benzyl-pipérazine, trichlorhydrate***

***EXEMPLE 100*** ***: N-(3,4-Diméthoxy-2-thioacétoxy)benzyl-N'-éthyl-pipérazine, dichlorhydrate***

A une solution de 10 mmoles de N-(2-acétoxy-3,4-diméthoxy)benzyl-N'-éthyl-pipérazine décrite dans l'exemple 5 dans 50 ml de xylène, sous atmosphère inerte, sont ajoutées, sous agitation, 12 mmoles du réactif de Lawesson. Le milieu réactionnel est porté à 130°C pendant 18heures. Après refroidissement, évaporation du solvant, le résidu est chromatographié sur gel de silice avec de l'éther de pétrole puis du dichlorométhane et conduit au produit attendu.

***EXEMPLE 101*** ***: N-(3,4-Diméthoxy-2-thioacétoxy)benzyl-N'-benzyl-pipérazine, dichlorhydrate***

Le produit attendu est obtenu selon le procédé décrit dans l'exemple 31.

**ETUDE PHARMACOLOGIOUE DES DERIVES DE L'INVENTION**

Les composés de l'invention ont été étudiés comparativement à la trimétazidine (2,3,4-triméthoxybenzyl pipérazine).

***EXEMPLE 102*** ***: Etude des composés de l'invention sur la fonction contractile du myocarde et sur la réponse métabolique énergétique myocytaire au cours d'une séquence ischémie-reperfusion (coeur isolé de rat).***

Les effets anti-ischémiques de la trimétazidine s'accompagnent, lors d'un essai effectué sur un coeur isolé de rat, d'une réduction de l'acidose ischémique et d'une protection des mécanismes producteurs d'ATP (LAVANCHY et col., *Advances in Studies on Heart Metabolism,* pp. 257-262, 1987). Cette étude était conduite grâce à l'utilisation

de la spectroscopie de RMN du $^{31}P$, technique qui permet le suivi, sur un coeur isolé et perfusé, de la variation des contenus en composés phosphorylés (ATP, phosphocréatine, phosphate inorganique) et du pH intracellulaire. Elle permet d'explorer en continu un certain nombre de variables liées à l'état énergétique du myocarde et d'évaluer d'une manière comparative les propriétés anti-ischémiques de plusieurs produits.

**Méthodes**

Le coeur isolé de rat est perfusé par voie aortique sous une pression constante (100 cm d'eau) durant 30 min., puis il est soumis à une ischémie globale partielle (débit coronaire réduit à 1,3 % du débit initial) durant 24 min., enfin, le coeur est reperfusé sous pression constante pendant 30 min.

Durant toute l'expérience, des spectres de RMN correspondant à des acquisitions d'une durée de 3 min. sont enregistrés. Après exploitation numérique, ils servent à déterminer la concentration intracardiaque de l'ATP, de la phosphocréatine (PC) et du phosphate inorganique (Pi), ainsi qu'à évaluer le pH intracellulaire. A partir de ces données, peuvent être déterminées les évolutions cinétiques de ces variables durant l'ischémie et au cours de la reperfusion.

**Protocole expérimental**

Ce protocole est destiné à apprécier l'effet de la présence du produit avant et durant l'ischémie. A cet effet, le produit est administré dans le milieu de perfusion après 20 min. de perfusion normoxique ; il reste présent dans la solution de perfusion durant le reste de l'expérience. La concentration de $5.10^{-6}$M a été choisie pour cette étude comparative.

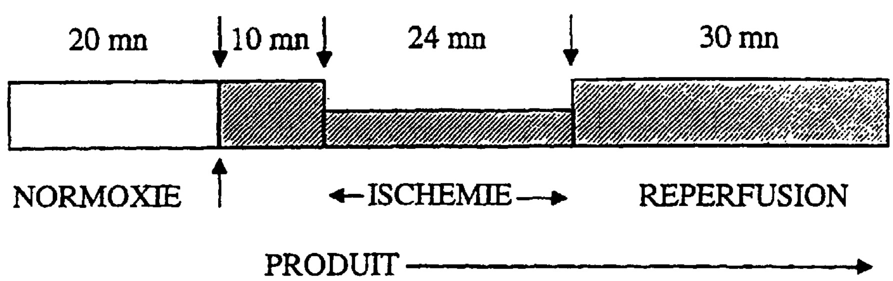


*1re série expérimentale :*

Elle est destinée à évaluer les effets des produits sur la fonction cardiaque. Le coeur est perfusé dans les conditions définies plus haut, et un catheter relié à un capteur de pression est introduit dans le ventricule gauche (via l'oreillette) afin de suivre les modifications de la pression développée par le VG. La fréquence cardiaque et le débit coronaire sont également suivis.

*2e série expérimentale :*

Elle est réalisée au sein d'un aimant supraconducteur de 5,9 T afin d'enregistrer des spectres de RMN du $^{31}P$. Cette série d'expériences est destinée à évaluer l'impact du produit sur l'équilibre énergétique du myocarde à la fois en normoxie, en ischémie et en reperfusion.

**Résultats**

Les résultats obtenus dans cette étude montrent que l'effet cytoprotecteur de la trimétazidine, lors d'une séquence ischémie-reperfusion du myocarde, se trouve être très augmenté avec les composés de l'invention et plus particulièrement avec le composé de l'exemple 1 qui exerce même un effet statistiquement significatif sur le contenu intracellulaire d'ATP. L'effet du composé de l'exemple 1 est également particulièrement net sur l'acidose intracellulaire induite par l'ischémie : le pH intracellulaire s'effondre de pH 7,2 à pH 6,0 en fin d'ischémie chez les groupes de contrôle, il est maintenu à pH 6,35 dans le groupe traité par la trimétazidine et à pH 6,50 dans le groupe traité par le composé de l'exemple 1, ce qui au plan physiopathologique est une différence considérable.

***EXEMPLE 103*** ***: Activité sur la production énergétique mitochondriale lors d'une surcharge calcique expérimentale***

La mitochondrie joue un rôle essentiel dans la production énergétique cellulaire sous forme d'ATP. La situation d'hypoxie cellulaire consécutive à une ischémie entraîne une surcharge calcique intracellulaire (MARBAN et coll., 80, 17-22, 1989) et en particulier mitochondriale, liée à la chute de synthèse d'ATP : il a été démontré que le stockage exagéré de calcium par la mitochondrie diminuait la synthèse d'ATP (TUENA de GOMEZ-PUYOU et coll., *Biochem. Biophys. Acta,* 532, 396-405, 1980) et que cet excès de calcium était à l'origine des désordres cellulaires caractéristiques des pathologies ischémiques (CHEUNG et coll., *New Engl. J. Med.,* 314, 1670-1676, 1986).
Cet effondrement de la synthèse mitochondriale d'ATP peut être créé expérimentalement sur des mitochondries isolées placées dans un milieu contenant de la cyclosporine A. Cette dernière bloque l'extrusion du $Ca^{2+}$ mitochondrial provoquant l'accumulation d'ions $Ca^{2+}$ dans la mitochondrie et une diminution de la phosphorylation oxydative productrice d'ATP (CROMPTON et coll., 1988 ; BROEKEMEIER et coll., *Biochem. J.,* 255, 357-360, 1989).

**Matériel et méthode**

Le protocole suivi est celui qui a été décrit par SALDUCCI et coll. *(J. Pharmacol. Exp. Ther.,* 277, 417-422, 1996). En bref, les mitochondries sont extraites de foies de rats. La respiration mitochondriale (1,5 mg de protéines placées en chambre de réaction) est provoquée par l'addition de succinate de Na, utilisé comme substrat (à $6.10^{-4}$M) et la phosphorylation oxydative est déclenchée par l'adjonction d'ADP au milieu de survie jusqu'à la concentration finale de $1.10^{-5}$M.
L'enregistrement des vitesses de consommation d'oxygène (en nmol/min./mg de protéine mitochondriale) permet le calcul des paramètres caractéristiques de la synthèse énergétique mitochondriale sous forme d'ATP.

**Résultats**

Les résultats obtenus dans cette étude montrent que les composés de l'invention restaurent de manière significative la synthèse mitochondriale d'ATP. Les composés de l'invention permettent une restauration comprise entre 80 et 100 %. A la concentration de $1.10^{-6}$M, 100 % de restauration ont été observés avec le composé de l'exemple 1 alors que cette restauration n'est que de 47 % avec la trimétazidine.

***EXEMPLE 104*** ***: Etude de l'aptitude de substances chimiques à franchir la barrière hémato-encéphalique***

**Description du modèle de barrière hémato-encéphalique**

Afin d'étudier "in vitro" les fonctions des capillaires cérébraux, nous avons mis au point un modèle de coculture qui permet de recréer la situation "in vivo". Les cellules endothéliales de capillaires et les astrocytes sont cultivés de part et d'autre d'un filtre.

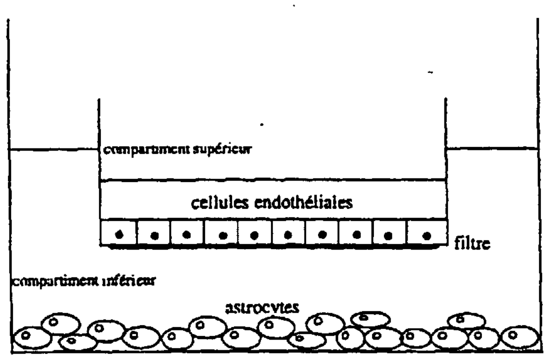


Les cellules endothéliales sont cultivées dans le compartiment supérieur sur le filtre et les astrocytes dans le compartiment inférieur sur le fond de la boîte de Pétri.
Dans ces conditions, les cellules retiennent tous les marqueurs de cellules endothéliales (présence du facteur VIII, surface non thrombogène, production de prostacycline, présence de l'enzyme de conversion) et de la barrière hémato-encéphalique (présence de jonctions serrées, rareté des vésicules de pinocytose, présence de monoamine oxydase

et γ-glutamyl-transpeptidase (Dehouck et col., *J. of Controlled Release.,* 21, 81-92, 1992).

**Méthode** (Dehouck et col., *J. of Controlled Release,* 21, 81-92, 1992).

Le jour de l'expérience, une solution de Ringer-HEPES (NaCl, 150 mM ; KCl, 5,2 mM ; $CaCl_2$, 2,2 mM ; $MgCl_2$ $6H_2O$, 0,2 mM ; $NaHCO_3$, 6 mM ; HEPES, 5 mM ; glucose 2,8 mM) est placée dans le compartiment inférieur d'une boîte à 6 puits (3 ml par puits). Un filtre recouvert d'une monocouche de cellules endothéliales est transféré dans le premier puits de la boîte à 6 puits. 2 ml de la solution de Ringer-HEPES contenant la molécule à tester sont placés dans le compartiment supérieur du filtre. Aux temps 10, 20, 30, 60, 90 et 120 minutes après addition de la molécule, les filtres sont transférés dans un autre puits de la boîte à 6 puits afin de réduire au maximum le passage possible de la molécule du compartiment inférieur au compartiment supérieur.
Les expériences sont réalisées en triplicate avec des filtres couverts d'une monocouche de cellules endothéliales de capillaires cérébraux ou de collagène seul comme témoin. Ceux-ci sont placés sous agitation et à 37°C tout au long de l'expérience.

**Analyse des résultats**

- Détermination du coefficient de perméabilité

Afin de déterminer les coefficients de perméabilité des molécules, la clearance de chaque substance est déterminée suivant la formule :

$$\text{clearance } (\mu l) = \text{Cl } (\mu l) = \frac{X}{Cd}$$

où X est la quantité de substance dans le compartiment inférieur,
où Cd est la concentration de substance dans le compartiment supérieur.

Pendant 60 minutes, la clearance augmente linéairement avec le temps. Sur le graphe clearance = f(t), la mesure de la pente nous donne la valeur "PSt" où PS = perméabilité x surface de la monocouche (en µl/min) pour les filtres recouverts de cellules endothéliales de capillaires cérébraux. Pour les filtres témoins "PSf' est calculée de la même façon. La perméabilité pour la monocouche de cellules endothéliales seule (PSe) est donnée par :

$$\frac{1}{PSe} = \frac{1}{PSt} - \frac{1}{PSf}$$

Le coefficient de perméabilité endothéliale "Pe" est calculé en divisant "PSe" par la surface de la monocouche de cellules endothéliales. "Pe" s'exprime en "cm/min".

**Conclusion**

La trimétazidine se caractérise par un coefficient de perméabilité de la BHE extrêmement faible (0,42) indiquant que la molécule pénètrent mal dans le cerveau et que son utilisation pour le traitement de pathologies cérébrales ne peut être envisagé.
Les composés de l'invention quant à eux présentent un coefficient de perméabilité de la BHE nettement plus important.
Les composés des exemples 1, 2, 17 et 19 en particulier qui présentent un coefficient de perméabilité de la BHE supérieur à 1, pénètrent au contraire très facilement dans le cerveau, de façon exactement proportionnelle à leur concentration dans le torrent circulatoire.

***EXEMPLE 105* : Composition pharmaceutique**

| Formule de préparation pour 1000 comprimés dosés à 10 mg | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

**1.** Composé de formule (I) :

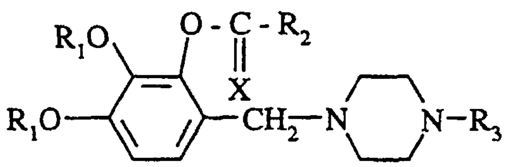

(I)

dans laquelle :

$R_1$ représente un groupement alkyle ($C_1$-$C_6$) linéaire ou ramifié,

X représente un atome d'oxygène ou de soufre,

$R_2$ représente un groupement alkyle ($C_1$-$C_8$) linéaire ou ramifié (éventuellement substitué par un groupement carboxy ou alkoxycarbonyle ($C_1$-$C_6$) linéaire ou ramifié), un groupement alkoxy ($C_1$-$C_6$) linéaire ou ramifié, un groupement phényle (éventuellement substitué par un ou plusieurs atomes d'halogène ou groupements alkyle ($C_1$-$C_6$) linéaire ou ramifié, alkoxy ($C_1$-$C_6$) linéaire ou ramifié, hydroxy ou trihalogénométhyle), un groupement cycloalkyle ($C_3$-$C_7$) (éventuellement substitué par un ou plusieurs groupements phényle eux-mêmes éventuellement substitués par un ou plusieurs atomes d'halogène ou groupements alkyle ($C_1$-$C_6$) linéaire ou ramifié, alkoxy ($C_1$-$C_6$) linéaire ou ramifié, hydroxy, ou trihalogénométhyle), un groupement 4-(2,3-dithiacyclopent-1-yl)butyl, un groupement pyridyle, un groupement amino (éventuellement substitué par un ou deux groupements alkyle ($C_1$-$C_6$) linéaire ou ramifié), ou l'un quelconque des groupements suivants :

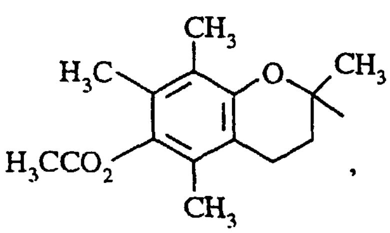

,

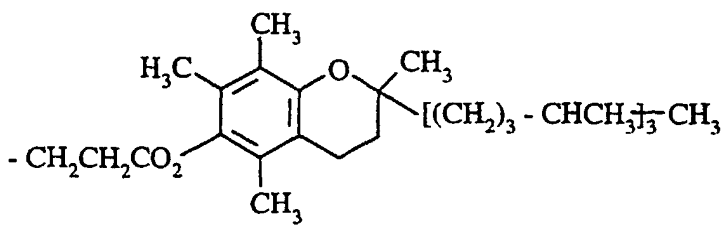

$R_3$ représente un atome d'hydrogène, un groupement cycloalkyle ($C_3$-$C_7$), un groupement formyle, un groupement phényle (éventuellement substitué par un ou plusieurs atomes d'halogène ou groupements alkyle ($C_1$-$C_6$) linéaire ou ramifié, alkoxy ($C_1$-$C_6$) linéaire ou ramifié, hydroxy ou trihalogénométhyle), un groupement pyridyle,
ou, un groupement alkyle ($C_1$-$C_{20}$) linéaire ou ramifié éventuellement substitué par un ou plusieurs, identiques ou différents, atomes d'halogène ou groupements :

- phényle éventuellement substitué par un plusieurs atomes d'halogène ou groupements alkyle ($C_1$-$C_6$) linéaire ou ramifié, alkoxy ($C_1$-$C_6$) linéaire ou ramifié, hydroxy ou trihalogénométhyle,
- cycloalkyle ($C_3$-$C_7$) éventuellement substitué par un ou plusieurs groupements phényle eux-mêmes éventuellement substitués par un ou plusieurs atomes d'halogène ou groupements alkyle ($C_1$-$C_6$) linéaire ou ramifié, alkoxy ($C_1$-$C_6$) linéaire ou ramifié, hydroxy ou trihalogénométhyle,
- alkoxy ($C_1$-$C_6$) linéaire ou ramifié,

- hydroxy,
- ou, pyrolidinyle,

leurs stéréoisomères lorsqu'ils existent ainsi que leurs sels d'addition à un acide ou à une base pharmaceutiquement acceptable.

2. Composé de formule (I) selon la revendication 1 tel que X représente un atome d'oxygène.

3. Composé de formule (I) selon la revendication 1 tel que X représente un atome de soufre.

4. Composé de formule (I) selon la revendication 1 tel que $R_1$ représente un groupement méthyle.

5. Composé de formule (I) selon la revendication 1 tel que $R_2$ représente un groupement cycloalkyle (C3-C7) éventuellement substitué par un ou plusieurs groupements phényle eux-mêmes éventuellement substitués.

6. Composé de formule (I) selon la revendication 4 tel que $R_2$ représente un groupement phénylcyclopropyle.

7. Composé de formule (I) selon la revendication 1 tel que $R_3$ représente un groupement alkyle éventuellement substitué.

8. Composé de formule (I) selon la revendication 1 qui est le N-(3,4-diméthoxy-2-E-phénylcyclopropylcarbonyloxy) benzyl-N'-éthyl-pipérazine, ses stéréoisomères ainsi que ses sels d'addition à un acide pharmaceutiquement acceptable.

9. Procédé de préparation des composés de formule (I) selon la revendication caractérisé en ce que l'on utilise comme produit de départ un composé de formule (II) :

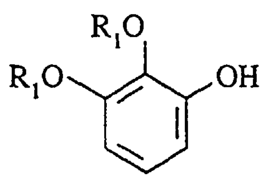

(II)

dans laquelle $R_1$ a la même signification que dans la formule (I), que l'on fait réagir avec une pipérazine substituée de formule (III) :

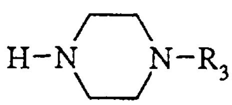

(III)

dans laquelle $R_3$ a la même signification que dans la formule (I), en présence de formaldéhyde,
pour conduire au composé de formule (IV) ;

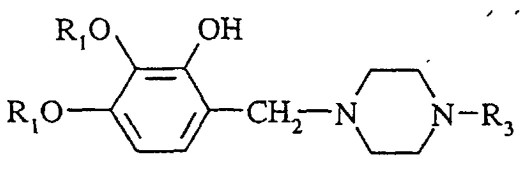

(IV)

dans laquelle $R_1$ et $R_3$ ont la même signification que dans la formule (I),
que l'on fait ensuite réagir avec un acide carboxylique de formule $R_2CO_2H$ (dans laquelle $R_2$ a la même signification que dans la formule (I), ou un chloroformiate d'alkyle ou bien encore un chlorure d'acide de formule $R_2COCl$ (dans laquelle $R_2$ a la même signification que précédemment),
pour conduire au composé de formule (I/a), cas particulier des composés de formule (I) :

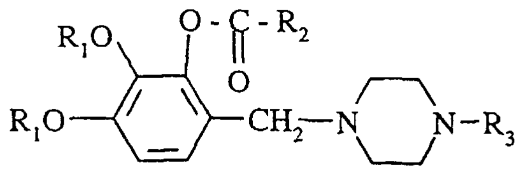

(I/a)

dans laquelle $R_1$, $R_2$ et $R_3$ ont la même signification que précédemment, composé de formule (I/a) que l'on soumet éventuellement à l'action du réactif de Lawesson, pour conduire au thioester correspondant de formule (I/b), cas particulier des composés de formule (I) :

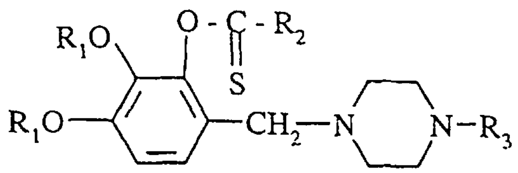

(I/b)

dans laquelle $R_1$, $R_2$ et $R_3$ ont la même signification que dans la formule (I), composés de formule (I/a) ou (I/b), que l'on purifie, le cas échéant, selon une technique classique de purification, dont on sépare éventuellement les stéréoisomères lorsqu'ils existent, et que l'on transforme, si on le souhaite, en sel d'addition à un acide ou une base pharmaceutiquement acceptable.

**10.** Compositions pharmaceutiques contenant comme principe actif au moins un composé selon l'une quelconque des revendications 1 à 8 seul ou en combinaison avec un ou plusieurs excipients ou véhicules inertes, non toxiques, pharmaceutiquement acceptables.

**11.** Compositions pharmaceutiques selon la revendication 10 contenant au moins un principe actif selon l'une quelconque des revendications 1 à 8 utiles pour le traitement de l'ischémie cellulaire chronique, des accidents ischémiques aigus cérébraux, cardiaques ou périphériques, pour le traitement des maladies neurodégénératives chroniques comme la maladie d'Alzheimer ou la maladie de Parkinson, ainsi que pour l'amélioration de la conservation d'organes destinés à des transplants et la survie des greffes lors du stress de reperfusion.

**Patentansprüche**

**1.** Verbindung der Formel (I):

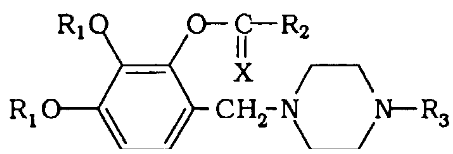

(I)

in der:

$R_1$ eine geradkettige oder verzweigte ($C_1$-$C_6$)-Alkylgruppe,

X ein Sauerstoffatom oder ein Schwefelatom,

$R_2$ eine geradkettige oder verzweigte (gegebenenfalls durch eine Carboxygruppe oder eine geradkettige oder verzweigte ($C_1C_6$)-Alkoxycarbonylgruppe substituierte) ($C_1$-$C_8$)-Alkylgruppe, eine geradkettige oder verzweigte ($C_1$-$C_6$)-Alkoxy-gruppe, eine (gegebenenfalls durch ein oder mehrere Halogenatome oder geradkettige oder verzweigte ($C_1C_6$)-Alkylgruppen, geradkettige oder verzweigte ($C_1$-$C_6$)-Alkoxygruppen, Hydroxygruppen oder Trihalogenmethylgruppen substituierte) Phenylgruppe, eine (gegebenenfalls durch eine oder mehrere Phenylgruppen, die ihrerseits gegebenenfalls durch ein oder mehrere Halogenatome oder geradkettige oder verzweigte ($C_1C_6$)-Alkylgruppen, geradkettige oder verzweigte ($C_1C_6$)-Alkoxygruppen, Hydroxy-

gruppen oder Trihalogenmethylgruppen substituiert sind, substituierte) ($C_3$-$C_7$)-Cycloalkylgruppe, eine 4-(2,3-Dithiacyclopent-1-yl)-butylgruppe, eine Pyridylgruppe, eine (gegebenenfalls durch eine oder zwei geradkettige oder verzweigte ($C_1$-$C_6$)-Alkylgruppen substituierte) Aminogruppe oder eine der folgenden Gruppen:

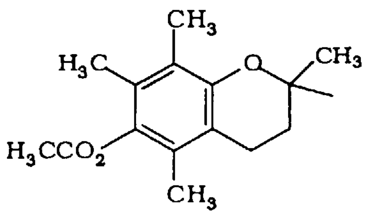

,

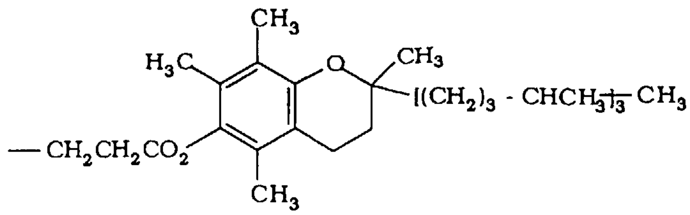

$R_3$ ein Wasserstoffatom, eine ($C_3$-$C_7$)-Cycloalkylgruppe, eine Formylgruppe, eine (gegebenenfalls durch ein oder mehrere Halogenatome oder geradkettige oder verzweigte ($C_1$-$C_6$)-Alkylgruppen, geradkettige oder verzweigte ($C_1$-$C_6$)-Alkoxy-gruppen, Hydroxygruppen oder Trihalogenmethylgruppen substituierte) Phenylgruppe, eine Pyridylgruppe oder eine geradkettige oder verzweigte ($C_1$-$C_{20}$)-Alkylgruppe, die gegebenenfalls durch ein oder mehrere, gleichartige oder verschiedene, Halogenatome oder Gruppen:

- Phenyl, gegebenenfalls substituiert durch ein oder mehrere Halogenatome oder geradkettige oder verzweigte ($C_1$-$C_6$)-Alkylgruppen, geradkettige oder verzweigte ($C_1$-$C_6$)-Alkoxygruppen, Hydroxygruppen oder Trihalogenmethylgruppen,
- ($C_3$-$C_7$)-Cycloalkyl, gegebenenfalls substituiert durch eine oder mehrere Phenylgruppen, die ihrerseits gegebenenfalls substituiert sind durch ein oder mehrere Halogenatome oder geradkettige oder verzweigte ($C_1$-$C_6$)-Alkyl-gruppen, geradkettige oder verzweigte ($C_1$-$C_6$)-Alkoxygruppen, Hydroxygruppen oder Trihalogenmethylgruppen,
- geradkettiges oder verzweigtes ($C_1$-$C_6$)-Alkoxy,
- Hydroxy

- oder Pyrrolidinyl substituiert ist, beteuten,
deren Stereoisomere, falls sie existieren, sowie deren Additionssalze mit einer pharmazeutisch annehmbaren Säure oder Base.

**2.** Verbindung der Formel (I) nach Anspruch 1, worin X ein Sauerstoffatom bedeutet.

**3.** Verbindung der Formel (I) nach Anspruch 1, worin X ein Schwefelatom bedeutet.

**4.** Verbindung der Formel (I) nach Anspruch 1, worin $R_1$ eine Methylgruppe bedeutet.

**5.** Verbindung der Formel (I) nach Anspruch 1, worin $R_2$ eine ($C_3$-$C_7$)-Cyclo-alkylgruppe, die gegebenenfalls substituiert ist durch eine oder mehrere Phenylgruppen, die ihrerseits gegebenenfalls substituiert sind, bedeutet.

**6.** Verbindung der Formel (I) nach Anspruch 4, worin $R_2$ eine Phenylcyclopro-pylgruppe bedeutet.

**7.** Verbindung der Formel (I) nach Anspruch 1, worin $R_3$ eine gegebenenfalls substituierte Alkylgruppe bedeutet.

**8.** Verbindung der Formel (I) nach Anspruch 1, nämlich N-(3,4-Dimethoxy-2-E-phenylcyclopropylcarbonyloxy)-benzyl-N'-ethyl-piperazin, dessen Stereoisomere sowie dessen Additionssalze mit einer pharmazeutisch annehmbaren Säure.

**9.** Verfahren zur Herstellung der Verbindungen der Formel (I) nach Anspruch 1, dadurch gekennzeichnet, daß man als Ausgangsprodukt eine Verbindung der Formel (II) verwendet:

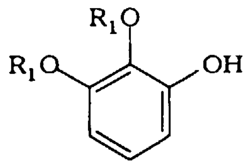

(II)

in der $R_1$ die bezüglich der Formel (I) angegebenen Bedeutungen besitzt, welche man in Gegenwart von Formaldehyd mit einem substituierten Piperazin der Formel (III) umsetzt:

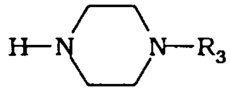

(III)

In der $R_3$ die bezüglich der Formel (I) angegebenen Bedeutungen besitzt,
zur Bildung der Verbindung der Formel (IV):

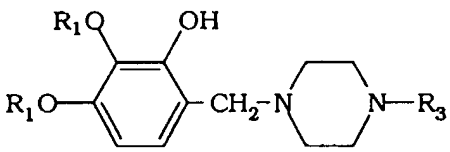

(IV)

in der $R_1$ und $R_3$ die bezüglich der Formel (I) angegebenen Bedeutungen besitzen, welche man anschließend mit einer Carbonsäure der Formel $R_2CO_2H$ (worin $R_2$ die bezüglich der Formel (I) angegebenen Bedeutungen besitzt, oder einem Chlorameisensäurealkylester oder schließlich einem Säurechlorid der Formel $R_2COCl$ (worin $R_2$ die oben angegebenen Bedeutungen besitzt) umsetzt),
zur Bildung der Verbindung der Formel (I/a), einem Sonderfall der Verbindungen der Formel (I):

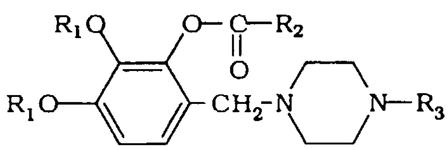

(I/a)

in der $R_1$, $R_2$ und $R_3$ die oben angegebenen Bedeutungen besitzen,
welche Verbindung der Formel (I/a) man gegebenenfalls der Einwirkung eines Lawesson-Reagens unterwirft zur Bildung des entsprechenden Thioesters der Formel (I/b), einem Sonderfall der Verbindungen der Formel (I):

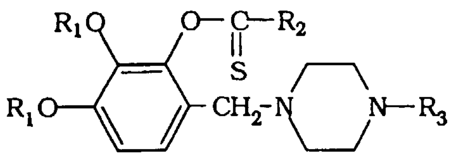

(I/b)

in der $R_1$, $R_2$ und $R_3$ die bezüglich der Formel (I) angegebenen Bedeutungen besitzen,
welche Verbindungen der Formeln (I/a) oder (I/b) man
gegebenenfalls mit Hilfe einer klassischen Reinigungsmethode reinigt, man gegebenenfalls in ihre Stereoisomeren, falls diese existieren, auftrennt und man gewünschtenfalls in ihre Additionssalze mit einer pharmazeutisch annehmbaren Säure oder Base überführt.

**10.** Pharmazeutische Zubereitungen enthaltend als Wirkstoff mindestens eine Verbindung nach einem der Ansprüche 1 bis 8 allein oder in Kombination mit einem oder mehreren inerten, nichttoxischen, pharmazeutisch annehmbaren Trägermaterialien oder Bindemitteln.

**11.** Pharmazeutische Zubereitungen nach Anspruch 10 enthaltend mindestens einen Wirkstoff nach einem der Ansprüche 1 bis 8 zur Behandlung von chronischer Zellischämie, akuten ischämischen Vorfällen des Gehirns, des Herzens und der peripheren Gefäße, zur Behandlung von chronischen neurodegenerativen Erkrankungen, wie der Alzheimerschen Krankheit oder der Parkinsonschen Krankheit, sowie zur Verbesserung der Aufbewahrung von für die Transplantation bestimmten Organen und des Überlebens von aufgepfropften Implantaten gegenüber dem Reperfusionsstress.

## Claims

**1.** Compound of formula (I):

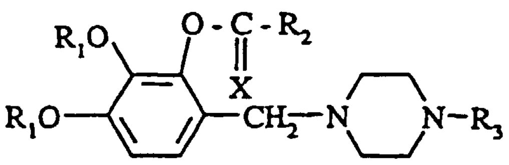

(I)

wherein:

$R_1$ represents a linear or branched ($C_1$-$C_6$)alkyl group,
X represents an oxygen or sulphur atom,
$R_2$ represents a linear or branched ($C_1$-$C_8$)alkyl group (optionally substituted by a carboxy group or a linear or branched ($C_1$-$C_6$)alkoxycarbonyl group), a linear or branched ($C_1$-$C_6$)alkoxy group, a phenyl group (optionally substituted by one or more halogen atoms or linear or branched ($C_1$-$C_6$)alkyl groups, linear or branched ($C_1$-$C_6$)-alkoxy groups, hydroxy groups or trihalomethyl groups), a ($C_3$-$C_7$)cydoalkyl group (optionally substituted by one or more phenyl groups which may themselves be substituted by one or more halogen atoms, linear or branched ($C_1$-$C_6$)alkyl groups, linear or branched ($C_1$-$C_6$)alkoxy groups, hydroxy groups or trihalomethyl groups), a 4-(2,3-dithiacyclopent-1-yl)butyl group, a pyridyl group, an amino group (optionally substituted by one or two linear or branched ($C_1$-$C_6$)alkyl groups) or any one of the following groups:

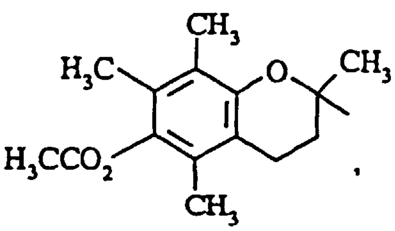

,

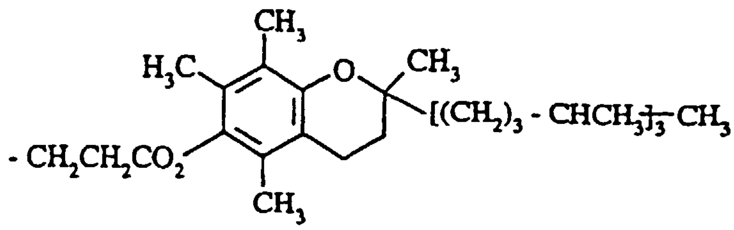

$R_3$ represents a hydrogen atom, a ($C_3$-$C_7$)cycloalkyl group, a formyl group, a phenyl group (optionally substituted by one or more halogen atoms, linear or branched ($C_1$-$C_6$)alkyl groups, linear or branched ($C_1$-$C_6$)alkoxy groups, hydroxy groups or trihalomethyl groups), a pyridyl group, or

a linear or branched ($C_1$-$C_{20}$)alkyl group optionally substituted by one or more, identical or different, halogen atoms or groups:

- phenyl optionally substituted by one or more halogen atoms, linear or branched ($C_1$-$C_6$)alkyl groups, linear or branched ($C_1$-$C_6$)alkoxy groups, hydroxy groups or trihalomethyl groups,
- ($C_3$-$C_7$)cycloalkyl optionally substituted by one or more phenyl groups which may themselves be substituted by one or more halogen atoms, linear or branched ($C_1$-$C_6$)alkyl groups, linear or branched ($C_1$-$C_6$)alkoxy groups, hydroxy groups or trihalomethyl groups,
- linear or branched ($C_1$-$C_6$)alkoxy,
- hydroxy, or
- pyrrolidinyl,

their stereoisomers, when they exist, and addition salts thereof with a pharmaceutically acceptable acid or base.

**2.** Compound of formula (I) according to claim 1 wherein X represents an oxygen atom.

**3.** Compound of formula (I) according to claim 1 wherein X represents a sulphur atom.

**4.** Compound of formula (I) according to claim 1 wherein $R_1$ represents a methyl group.

**5.** Compound of formula (I) according to claim 1 wherein $R_2$ represents a ($C_3$-$C_7$)-cycloalkyl group optionally substituted by one or more phenyl groups which may themselves be substituted.

**6.** Compound of formula (I) according to claim 4 wherein $R_2$ represents a phenylcyclopropyl group.

**7.** Compound of formula (I) according to claim 1 wherein $R_3$ represents an optionally substituted alkyl group.

**8.** Compound of formula (I) according to claim 1 that is N-(3,4-dimethoxy-2-E-phenylcyclopropylcarbonyloxy)benzyl-N'-ethylpiperazine, its stereoisomers and addition salts thereof with a pharmaceutically acceptable acid.

**9.** Process for the preparation of compounds of formula (I) according to claim 1, characterised in that there is used as starting material a compound of formula (II):

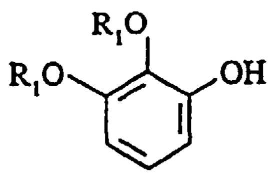

(II)

wherein $R_1$ is as defined for formula (I), which is reacted with a substituted piperazine of formula (III):

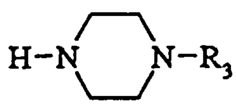

(III)

wherein $R_3$ is as defined for formula (I), in the presence of formaldehyde,
to yield a compound of formula (IV):

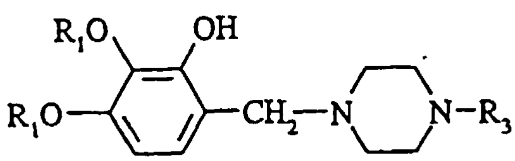

(IV)

wherein $R_1$ and $R_3$ are as defined for formula (I),
which is then reacted with a carboxylic acid of formula $R_2CO_2H$ (wherein $R_2$ is as defined for formula (I)), or an alkyl chloroformate or an acid chloride of formula $R_2COCl$ (wherein $R_2$ is as defined hereinbefore),
to yield a compound of formula (I/a), a particular case of the compounds of formula (I):

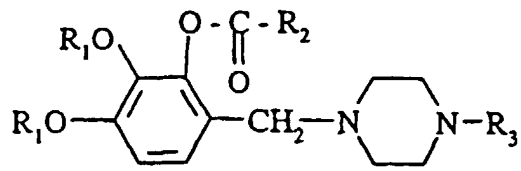

(I/a)

wherein $R_1$, $R_2$ and $R_3$ are as defined hereinbefore, which compound of formula (I/a) is optionally subjected to the action of Lawessons' reagent, to yield the corresponding thioester of formula (I/b), a particular case of the compounds of formula (I):

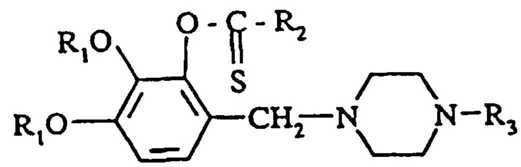

(I/b)

wherein $R_1$, $R_2$ and $R_3$ are as defined for formula (I), which compounds of formulae (I/a) and (I/b) are purified, if necessary, according to a conventional purification technique, are separated, where appropriate, into their stereoisomers, when they exist, and converted, if desired, into addition salts with a pharmaceutically acceptable acid or base.

**10.** Pharmaceutical compositions comprising as active ingredient at least one compound according to any one of claims 1 to 8 alone or in combination with one or more inert, non-toxic, pharmaceutically acceptable excipients or carriers.

**11.** Pharmaceutical compositions according to claim 10 comprising at least one active ingredient according to any one of claims 1 to 8 for use in the treatment of chronic cellular ischaemia, acute cerebral, cardiac or peripheral ischaemic accidents, in the treatment of chronic neurodegenerative diseases, such as Alzheimer's disease or Parkinson's disease, and in improving the storage of organs for transplant and in the survival of the transplants in the event of reperfusion stress.